# EUROPEAN PATENT SPECIFICATION

(11) **EP 3 426 342 B1**
(45) Date of publication and mention of the grant of the patent: **08.04.2020**
(21) Application number: 17703447.7
(22) Date of filing: 10.02.2017
(51) Int. Cl.: A61N 5/10, A61B 6/00, A61B 5/055, G01R 33/48, G01R 33/56

(54) **SYNTHETIC COMPUTED TOMOGRAPHY IMAGING**
SYNTHETISCHE COMPUTERTOMOGRAPHIEBILDGEBUNG
IMAGERIE PAR TOMODENSITOMÉTRIE SYNTHÉTIQUE

(30) Priority: 10.03.2016 EP 16159577
(43) Date of publication of application: 16.01.2019
(73) Proprietor: Koninklijke Philips N.V., 5656 AG Eindhoven (NL)
(72) Inventor: WEISS, Steffen, 5656 AE Eindhoven (NL)
(74) Representative: Groenendaal, Greetje
(86) International application number: PCT/EP2017/053008
(87) International publication number: WO 2017/153122

(56) References cited:
- WO-A1-2015/144540
- CHRISTOPHER M RANK ET AL: "MRI-based treatment plan simulation and adaptation for ion radiotherapy using a classification-based approach", RADIATION ONCOLOGY, BIOMED CENTRAL LTD, LO, vol. 8, no. 1, 6 March 2013 (2013-03-06), page 51, XP021155728, ISSN: 1748-717X, DOI: 10.1186/1748-717X-8-51
- JUHA KORHONEN ET AL: "Influence of MRI-based bone outline definition errors on external radiotherapy dose calculation accuracy in heterogeneous pseudo-CT images of prostate cancer patients", ACTA ONCOLOGICA., vol. 53, no. 8, 7 July 2014 (2014-07-07), pages 1100-1106, XP055283550, GB ISSN: 0284-186X, DOI: 10.3109/0284186X.2014.929737
- MIKA KAPANEN ET AL: "T1/T2*-weighted MRI provides clinically relevant pseudo-CT density data for the pelvic bones in MRI-only based radiotherapy treatment planning", ACTA ONCOLOGICA., vol. 52, no. 3, 19 June 2012 (2012-06-19), pages 612-618, XP055283512, GB ISSN: 0284-186X, DOI: 10.3109/0284186X.2012.692883

## Description

### FIELD OF THE INVENTION

The invention relates to magnetic resonance imaging, in particular the construction of synthetic computed tomography images from magnetic resonance imaging data.

### BACKGROUND OF THE INVENTION

Synthetic or virtual Computed Tomography (CT) images are simulated CT images calculated using data from one or more other medical imaging modalities. The journal article Hsu, Shu-Hui et al. "Investigation of a Method for Generating Synthetic CT Models from MRI Scans of the Head and Neck for Radiation Therapy." Physics in medicine and biology 58.23 (2013): 10.1088/0031-9155/58/23/8419. PMC. Web. 14 Oct. 2015 describes one method of generating virtual CT images.

The journal article Cocosco et. al., "A fully automatic and robust brain MRI tissue classification method," Medical Image Analysis 7 (2003) 513-527 describes a method of identifying different tissues types of brain tissues using an Magnetic Resonance Image (MRI).

The journal article Korhonen et. al., "A dual model HU conversion from MRI intensity values within and outside of bone segment for MRI -based radiotherapy treatment planning of prostate cancer, Med. Phys. 41, 011704 (2014) DOI: 10.1118/1.4842575 describes a method of converting MRI intensity into Hounsfield units (HUs) in the male pelvis. The model used was tuned for two patient-specific adjustments: MR signal normalization to correct shifts in absolute intensity level and application of a cutoff value to accuratels represent low density bony tissue HUs.

### SUMMARY OF THE INVENTION

The invention provides for a computer program product, a medical imaging method, and a medical system in the independent claims. Embodiments are given in the dependent claims.

Examples as described herein may possibly provide for the improved construction of synthetic CT images and/or improved radiotherapy planning by providing a means of providing for a personalized estimate of the Hounsfield value for cortical bone. Bone mineralization density data descriptive of a subject may be used to derive or calculate the personalized estimate of the Hounsfield value. An alternative to personalizing the Hounsfield value of cortical bone is to use a Hounsfield value for cortical bone which is the result of averaging a large number of subjects. However, personalizing the Hounsfield value of cortical bone for each subject may be beneficial because, cortical bone is one of the denser portions of the subject. Using an average value of the Hounsfield value for cortical bone which is not specific to a particular subject may therefore result in errors in the synthetic CT image. Correcting the Hounsfield value for cortical bone for a particular subject may therefore result in synthetic CT images which provide more accurate information to a doctor and/or may enable more accurate radiotherapy planning.

Magnetic Resonance (MR) data is defined herein as being the recorded measurements of radio frequency signals emitted by atomic spins using the antenna of a Magnetic resonance apparatus during a magnetic resonance imaging scan. Magnetic resonance data is an example of medical image data. A Magnetic Resonance Imaging (MRI) image is defined herein as being the reconstructed two or three dimensional visualization of anatomic data contained within the magnetic resonance imaging data. This visualization can be performed using a computer.

A synthetic CT image as used herein refers to a simulated or modeled computed tomography (CT) image that has been calculated using medical imaging data of a different imaging modality. The term synthetic CT image is also synonymous with pseudo CT image, which is also sometimes used in the literature. Within this application, a synthetic CT image refers specifically to a synthetic CT image calculated using magnetic resonance data and/or one or more magnetic resonance images. A single MR image can be used to calculate a synthetic CT image, however various mappings of different MR parameters such as the T1 relaxation time, T2 relaxation time, proton density, and other values can be used to refine the mapping of MR data and or images into Hounsfield units (HUs). The reference of a magnetic resonance image in the description and the claims is therefore to be understood as possibly referring to multiple images and maps of various MR parameters.

The term Hounsfield unit as used herein encompasses a quantitative scale representing the radiodensity of an object or a portion of an object. The term Hounsfield unit is interchangeable with the terms HU, Hounsfield units, Hounsfield unit, Hounsfield scale, and CT number. A Hounsfield unit value is a value assigned to a voxel or region that is given in Hounsfield units.

In one aspect the invention provides for a computer program product for calculating a synthetic CT image. A synthetic CT image as used herein encompasses a medical image, which has been constructed to resemble the results of a CT or computer tomography scan. However, this synthetic CT image is made from a different imaging modality. The computer program product comprises machine-executable instructions for execution by a processor.

Execution of the machine-executable instructions causes the processor to receive bone mineralization density data. Bone mineralization density data as used herein encompasses data which is descriptive of the bone mineralization of bones of a subject. Bone mineralization density data may be acquired in the course of determining if a subject has low bone density. For example hospitals particularly are able to perform tests on bone mineralization to diagnose people with osteoporosis. As used herein bone mineralization density data may encompass data acquired by an apparatus for measuring bone mineralization. This may be typically done with X-rays or other ionizing radiation. Execution of the machine-executable instructions further causes the processor to receive a magnetic resonance image of a region of interest. Execution of the machine-executable instructions further causes the processor to calculate an image segmentation by segmenting the magnetic resonance image into a set of tissue types. The set of tissue types comprises a cortical bone segmentation. There exist standard imaging segmentation techniques for segmenting a magnetic resonance image and dividing it into a variety or set of tissue types. Execution of the machine-executable instructions further cause the processor to calculate a cortical bone Hounsfield value using the bone mineralization density data. The so called Hounsfield unit or Hounsfield unit scale may also be referred to as a CT number. A Hounsfield unit is a quantitative scale for describing the radio density of a particular tissue type. The Hounsfield unit value is a measure of the absorption of ionizing radiation such as X-rays. By calculating a cortical bone Hounsfield value the magnetic resonance image may be used to more accurately produce the synthetic CT image.

Execution of the machine-executable instructions further cause the processor to calculate a Hounsfield unit mapping by assigning at least one Hounsfield unit value to each of the set of tissue types in the image segmentation. The cortical bone segmentation is assigned the cortical bone Hounsfield value. The creation of the Hounsfield unit mapping may be performed in several different ways. In the simplest version, the tissue types may be identified and the Hounsfield unit values may simply be assigned to those regions. In other examples the tissue region may be identified and then other parameters such as a parameter measured by one or more magnetic resonance images may be used to assign the Hounsfield unit value. For example, a tissue type may be first identified and then MR parameters such as the T1, T2 relaxation time, proton density or other values may be used to determine the Hounsfield Unit mapping within each particular tissue type. As described herein the cortical bone segmentation is assigned the cortical bone Hounsfield value that was calculated by the processor. Execution of the machine-executable instructions further cause the processor to calculate the synthetic CT image using the Hounsfield unit mapping. If the Hounsfield unit mapping has been accurately calculated or estimated then a reasonably accurate synthetic CT image may be generated by modeling the transmission of radiation through the region of interest imaged by the magnetic resonance image.

This embodiment may be beneficial because the regions of the cortical bone within the subject have been assigned a more accurate value of the Hounsfield unit mapping. This example may provide for a more accurately calculated synthetic CT image and/or may enable more accurate radiotherapy planning.

In another embodiment the bone mineralization density data is the bone mineralization density data of a subject. The magnetic resonance image of the region of interest in this embodiment is also for the same subject as the bone mineralization density data.

In another embodiment the medical system comprises a radiotherapy unit.

In another embodiment execution of the machine-executable instructions further cause the processor to receive radiation therapy treatment data that is at least partially descriptive of the region of interest. For example the radiation therapy treatment data may be data provided to the processor or may be received via a user interface which details a region which is intended to be treated with a radiation therapy device and also to delineate regions of the subject which is desired to minimize the amount of received radiation such as critical organs or other anatomical regions of a subject. Execution of the machine-executable instructions further cause the processor to calculate radiotherapy control instructions using at least the synthetic CT image and the radiotherapy treatment data. The synthetic CT image may be used to create a mapping of radiation absorption so that the radiation absorbed by a treatment region and also by portions of the subject outside of the treatment region can be modeled accurately. This may enable more accurate delivery of radiation to a treatment region or target zone and to minimize the amount of radiation received by critical organs.

In another embodiment the bone mineralization density data comprises dual energy X-ray absorptiometry data. When performing bone mineralization density measurements to diagnose osteoporosis a dual energy X-ray absorptiometry machine may be available. This embodiment may be beneficial because it repurposes the data typically used for detecting osteoporosis to improve the quality of synthetic CT images.

In another embodiment the calculating of the cortical bone Hounsfield value comprises determining a dual energy X-ray absorptiometry bone density measurement value from the dual energy X-ray absorptiometry data. The cortical bone Hounsfield value is determined by applying an empirical transfer function to a dual energy X-ray absorptiometry bone density measurement value. For example a formula or lookup table could be used to translate the dual energy X-ray absorptiometry bone density measurement value into the cortical bone Hounsfield value. In other examples this could be refined further. For example the health status, the gender, the age, and other factors may be related to the correlation between the cortical bone density and the measured dual energy X-ray absorptiometry bone density measurement value. This additional data could be used for selecting a particular empirical transfer function to improve the quality of the cortical bone Hounsfield value that is provided.

The construction of an empirical transfer function may be straight forward. For a number of test subjects the measurement of the dual energy X-ray absorptiometry bone density measurement could be complimented by measurement using a CT system which can measure the cortical bone density correctly. Such measurements for a large number of test subjects with different gender, age, health and weight conditions may be used to generate an accurate empirical transfer function.

In another embodiment the dual energy X-ray absorptiometry data comprises a dual energy X-ray absorptiometry image. Calculating the cortical bone Hounsfield value in this case comprises first locating a cortical bone region in the dual energy X-ray absorptiometry image by segmenting the dual energy X-ray absorptiometry image. For example a simple model of a bone could be made and then only the cortical bone region or region which contains a large number of cortical bone can be selected. The calculation of the cortical bone Hounsfield value further comprises determining a cortical bone density from the cortical bone region. The calculation of the cortical bone Hounsfield value further comprises assigning the cortical bone Hounsfield value using the cortical bone density and a lookup table. In this case the measurement, though it is not a direct measurement, is taking data only from a region which contains mostly cortical bone. This enables a more accurate determination of the cortical bone Hounsfield value and also may free it from dependency upon such values as the gender, age, weight and other health characteristics.

In another embodiment the bone mineralization density data comprises a quantitative computer tomography image. Execution of the instructions further cause the processor to locate a cortical bone region in the quantitative computer tomography image by segmenting the quantitative computer tomography image. Execution of the instructions further cause the processor to assign the cortical bone Hounsfield value using the cortical bone region in the quantitative computer tomography image. This embodiment may be beneficial because the bone mineralization density data may be directly calculated from the quantitative computer tomography image. This may be performed in several different ways. In one case the bone mineralization density data is calculated first and then this is used to translate into the cortical bone Hounsfield value. As a measurement is performed with a CT system the measurement may also be translated directly into the Hounsfield value from the CT value determined by the quantitative computer tomography image.

In another aspect the invention provides for a medical imaging method. The method comprises receiving bone mineralization density data. The method further comprises receiving a magnetic resonance image of a region of interest. The method further comprises calculating an image segmentation by segmenting the magnetic resonance image into a set of tissue types. The set of tissue types comprises a cortical bone segmentation. The method further comprises calculating a cortical bone Hounsfield value using the bone mineralization density data. The method further comprises calculating a Hounsfield unit mapping by assigning at least one Hounsfield unit value to each of the set of tissue types in the image segmentation. The cortical bone segmentation is assigned the cortical bone Hounsfield value. The method further comprises calculating a synthetic CT image using the Hounsfield unit mapping.

In another aspect the invention provides for a medical system. The medical system comprises a memory for storing machine-executable instructions. The medical system further comprises a processor for controlling the medical system. Execution of the machine-executable instructions causes the processor to receive bone mineralization density data. Execution of the machine-executable instructions further causes the processor to receive a magnetic resonance image of a region of interest. Execution of the machine-executable instructions further cause the processor to calculate an image segmentation by segmenting the magnetic resonance image into a set of tissue types. The set of tissue types comprises a cortical bone segmentation.

Execution of the machine-executable instructions further causes the processor to calculate a cortical bone Hounsfield value using the bone mineralization density data. Execution of the machine-executable instructions further causes the processor to calculate a Hounsfield unit mapping by assigning at least one Hounsfield unit value to each of the set of tissue types in the image segmentation. The cortical bone segmentation is assigned the cortical bone Hounsfield value. Execution of the machine-executable instructions further causes the processor to calculate the synthetic CT image using the Hounsfield unit mapping.

In another embodiment, the medical system further comprises a magnetic resonance imaging system. Execution of the machine-executable instructions further cause the processor to receive a magnetic resonance image of a region of interest by controlling the magnetic resonance imaging system to acquire magnetic resonance data from at least the region of interest. Execution of the machine-executable instructions further cause the processor to receive a magnetic resonance image by reconstructing the magnetic resonance image from the magnetic resonance imaging system.

In another embodiment, the medical system further comprises a radiotherapy unit. A radiotherapy unit as used herein encompasses an apparatus assigned for delivering ionizing radiation to a target region within the subject. For example the radiotherapy unit may be a barium system which uses a barium radioactive source to direct ionizing radiation at a target region within a subject. Other sorts of radiation are also possible. For example the radiotherapy unit may shoot charged particles such as those that come from a Linac at the target region.

Execution of the machine-executable instructions further causes the processor to receive radiation therapy treatment data that is at least partially descriptive of the region of interest. Execution of the machine-executable instructions further causes the processor to calculate the radiotherapy control instructions for controlling the radiotherapy unit using at least the synthetic CT image and the radiotherapy treatment data. Using the synthetic CT image to help to calculate the radiotherapy control instructions may be beneficial because the increased accuracy of the synthetic CT image may increase the accuracy to which the radiotherapy control instructions are calculated to deliver radiation to the target zone and to minimize ionizing radiation delivered to other portions of the subject. In another example execution of the machine-executable instructions further cause the processor to control the radiotherapy unit with the radiotherapy control instructions.

In another embodiment, the medical system further comprises an emission computer tomography system. Emission computer tomography systems may encompass systems that perform medical imaging by detecting the emission of radioactive compounds within the subject. For example a positron emission tomography or PET system is one example. A single photon emission computer tomography or SPECT is another example of an emission computer tomography system. Execution of the machine-executable instructions further causes the processor to control the emission computer tomography system to acquire gamma ray emission data. Execution of the machine-executable instructions further causes the processor to calculate a gamma ray absorption map using the synthetic CT image. Execution of the machine-executable instructions further causes the processor to reconstruct an emission computer tomography image using at least the gamma ray emission data and the gamma ray absorption map. As the cortical bone is relatively dense, calculating the density is thereby a more accurate Hounsfield unit or CT value may allow more accurate reconstruction of the emission computer tomography image.

In another embodiment the imaging region of the emission computer tomography system and the imaging zone of the magnetic resonance imaging system are co-registered.

In another embodiment the imaging region of the emission computer tomography system and the imaging zone of the magnetic resonance imaging system at least partially overlap.

In another embodiment the medical system further comprises a dual energy X-ray absorptiometry unit. The radiation therapy treatment data comprises a dual energy X-ray absorptiometry image. The cortical bone Hounsfield value is determined by applying an empirical transfer function to the dual energy X-ray absorptiometry bone density measurement value.

In another embodiment, calculating the cortical bone Hounsfield value comprises locating a cortical bone region in the dual energy X-ray absorptiometry image by segmenting the dual energy X-ray absorptiometry image. The calculation of the cortical bone Hounsfield value further comprises determining a cortical bone density from the cortical bone region. Calculating the cortical bone Hounsfield value further comprises assigning the cortical bone Hounsfield value using the cortical bone density and a lookup table.

In another embodiment, the medical system further comprises a quantitative computer tomography unit. The processor receives the bone mineralization density data from the quantitative computer tomography unit. The bone mineralization density data comprises a quantitative computer tomography image.

In another embodiment execution of the machine-executable instructions further cause the processor to locate a cortical bone region in the quantitative computer tomography image by segmenting the quantitative computer tomography image. Execution of the machine-executable instructions further causes the processor to assign the cortical bone Hounsfield value using the cortical bone region in the quantitative computer tomography image.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances the computer executable code may be in the form of a high level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks. A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

### BRIEF DESCRIPTION OF THE DRAWINGS

In the following preferred embodiments of the invention will be described, by way of example only, and with reference to the drawings in which:
Fig. 1 illustrates an example of a medical system;
Fig. 2 illustrates an example of a method of using the medical system of Fig. 1;
Fig. 3 illustrates a further example of a medical system;
Fig. 4 illustrates an example of a method of using the medical system of Fig. 3;
Fig. 5 illustrates a further example of a medical system;
Fig. 6 illustrates an example of a method of using the medical system of Fig. 5;
Fig. 7 illustrates a further example of a medical system;
Fig. 8 illustrates an example of a method of using the medical system of Fig. 7;
Fig. 9 illustrates a further example of a medical system; and
Fig. 10 shows a sketch of a femoral bone to illustrate different ways in which bone mineralization density data can be determined using different examples of the medical system of Fig. 9.

### DETAILED DESCRIPTION OF THE EMBODIMENTS

Like numbered elements in these Figs. are either equivalent elements or perform the same function. Elements which have been discussed previously will not necessarily be discussed in later Figs. if the function is equivalent.

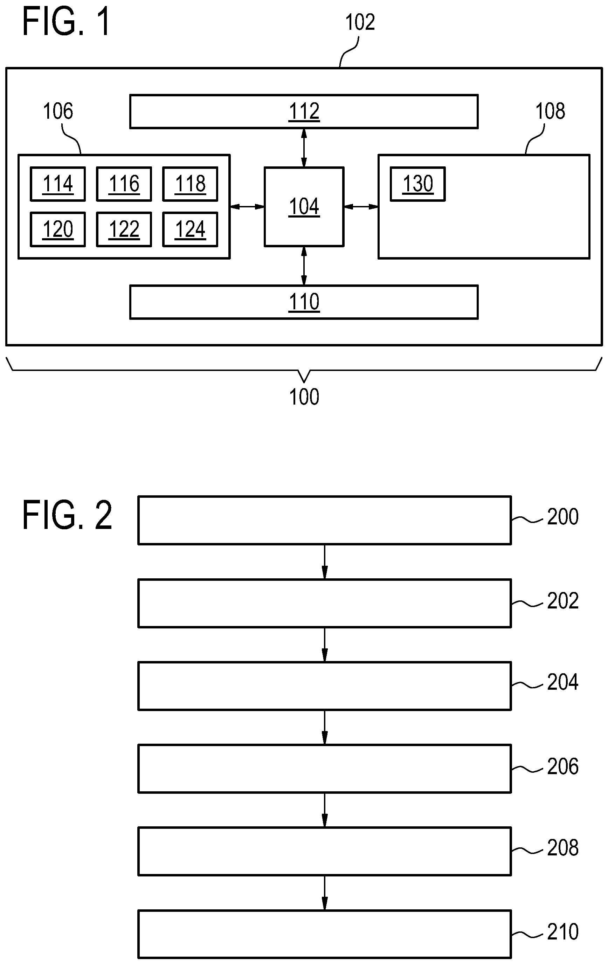

Fig. 1 shows an example of a medical system 100. The medical system 100 is shown as comprising a computer 102. The computer comprises a processor 104. The processor 104 is in communication with computer storage 106 and computer memory 108. The contents of the computer storage 106 and the computer memory 108 may duplicate each other. That is to say that the contents of the computer storage 106 may be interchanged with the contents of the computer memory 108. Because a particular element is shown in either the computer storage 106 or the computer memory 108 does not exclude it from being present in other variations. The processor 104 is further shown as being connected to a user interface 110. The processor 104 is shown as being in further contact with a hardware interface 112. In some examples there may be a hardware interface 112 which enables the processor 104 to control external components and to receive data.

The computer storage 106 is shown as containing bone mineralization density data 114. This for example may be received via the hardware interface 112 or may be received via user interface 110. The computer storage 106 is further shown as containing a magnetic resonance image 116. The computer storage 106 is shown as containing an image segmentation 118 of the magnetic resonance image 118. The computer storage 106 is further shown as containing a cortical bone Hounsfield value 120. The cortical bone Hounsfield value 120 is calculated from the bone mineralization density data 114. The computer storage 106 is further shown as containing a Hounsfield unit mapping 122 that was calculated from the image segmentation 118. In some examples the Hounsfield unit mapping 122 may be calculated from the image segmentation 118 and also from other data which is contained within the magnetic resonance image 116. The computer storage 106 is further shown as containing a synthetic CT image 124 that was calculated using the Hounsfield unit mapping 122.

The computer memory 108 is shown as containing machine-executable instructions 130 which enable the processor 104 to control the operation and function of the medical system 100 as well as to perform various numerical and calculating tasks.

Fig. 2 shows a flowchart which illustrates a method of operating the medical system 100 of Fig. 1. For example the flowchart of Fig. 2 may illustrate the steps or at least a portion of the steps that the machine-executable instructions 130 cause the processor 104 to perform. First in step 200 the bone mineralization density data 114 is received. Next in step 202 a magnetic resonance image of a region of interest is received. Next in step 204 the image segmentation 118 of the magnetic resonance image 116 is calculated. The image segmentation segments the magnetic resonance image into a set of tissue types. The set of tissue types comprise a cortical bone segmentation. Next in step 206 a cortical bone Hounsfield value 120 is calculated. The cortical bone Hounsfield value is at least partially calculated using the bone mineralization density data 114. Next in step 208 the Hounsfield unit mapping 122 is calculated by assigning at least one Hounsfield unit value to each of the set of tissue types in the image segmentation 118. The cortical bone segmentation is assigned the cortical bone Hounsfield value. Finally in step 210 the synthetic CT image 124 is calculated using the Hounsfield unit mapping 122.

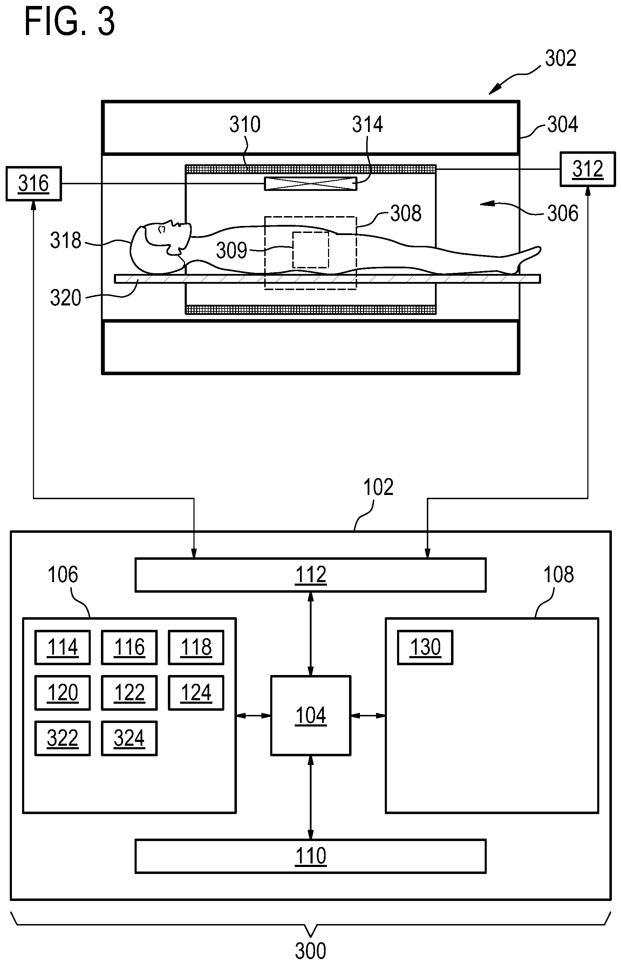

Fig. 3 shows a further example of a medical system 300. The medical system 300 shown in Fig. 3 is similar to that shown in Fig. 1 except the system now also includes a magnetic resonance imaging system 302. The magnetic resonance imaging system 302 comprises a magnet 304. The magnet 304 is a superconducting cylindrical type magnet with a bore 306 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 306 of the cylindrical magnet 304 there is an imaging zone 308 where the magnetic field is strong and uniform enough to perform magnetic resonance imaging.

Within the bore 306 of the magnet there is also a set of magnetic field gradient coils 310 which is used for acquisition of magnetic resonance data to spatially encode magnetic spins within the imaging zone 308 of the magnet 304. The magnetic field gradient coils 310 connected to a magnetic field gradient coil power supply 312. The magnetic field gradient coils 310 are intended to be representative. Typically magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio-frequency coil 314 for manipulating the orientations of magnetic spins within the imaging zone 308 and for receiving radio transmissions from spins also within the imaging zone 308. Using pulse sequence commands a sub set or region of interest 309 of the imaging zone 308 can be imaged. The radio frequency antenna may contain multiple coil elements. The radio frequency antenna may also be referred to as a channel or antenna. The radio-frequency coil 314 is connected to a radio frequency transceiver 316. The radio-frequency coil 314 and radio frequency transceiver 316 may be replaced by separate transmit and receive coils and a separate transmitter and receiver. It is understood that the radio-frequency coil 314 and the radio frequency transceiver 316 are representative. The radio-frequency coil 314 is intended to also represent a dedicated transmit antenna and a dedicated receive antenna. Likewise the transceiver 316 may also represent a separate transmitter and receivers. The radio-frequency coil 314 may also have multiple receive/transmit elements and the radio frequency transceiver 316 may have multiple receive/transmit channels. For example if a parallel imaging technique such as SENSE is performed, the radio-frequency could 314 will have multiple coil elements.

The transceiver 316 and the magnetic field gradient coil power supply 312 are all connected to a hardware interface 328 of computer system 326.

The computer storage is shown as additionally containing pulse sequence commands 322. Pulse sequence commands 322 as used herein are commands which can be used to directly control the magnetic resonance imaging system 302 or other data which may be converted into such commands which enable the magnetic resonance imaging system 302 to acquire magnetic resonance data. The computer storage 106 is further shown as containing magnetic resonance data 324 that has been acquired from the subject 318 for at least a region of interest 309 within the imaging zone 308 using the pulse sequence commands 322. The region of interest to which the magnetic resonance image 116 comes from may be any subset of imaging zone 308.

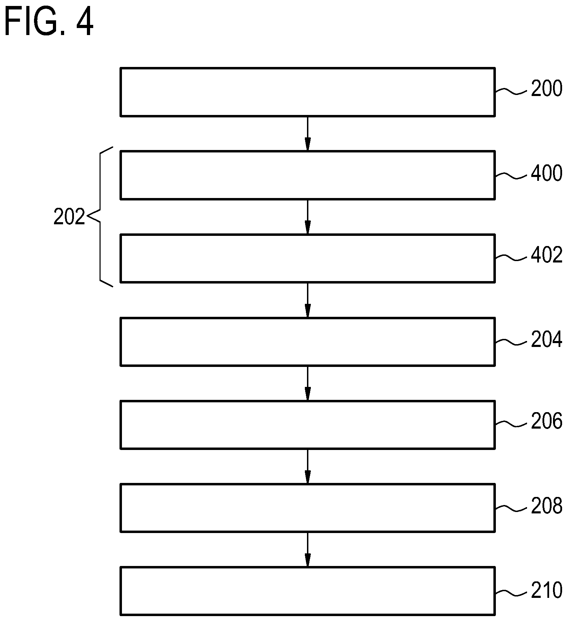

Fig. 4 shows a flowchart which illustrates a method of operating the medical system 300 of Fig. 3. The flowchart in Fig. 4 is similar to that shown in Fig. 2 except step 202 has been replaced by steps 400 and 402. Steps 400 and 402 illustrate one particular example of how the magnetic resonance image may be received. In this case the magnetic resonance imaging system 302 is used to acquire magnetic resonance data which is then reconstructed by the processor 104. After step 200 is performed step 400 is performed. In step 400 the magnetic resonance imaging system is controlled by the processor 104 to acquire magnetic resonance data 324 from at least the region of interest. Next in step 402 magnetic resonance image 116 is reconstructed from the magnetic resonance data 324. Next the method then proceeds onto step 204 as is shown in Fig. 2.

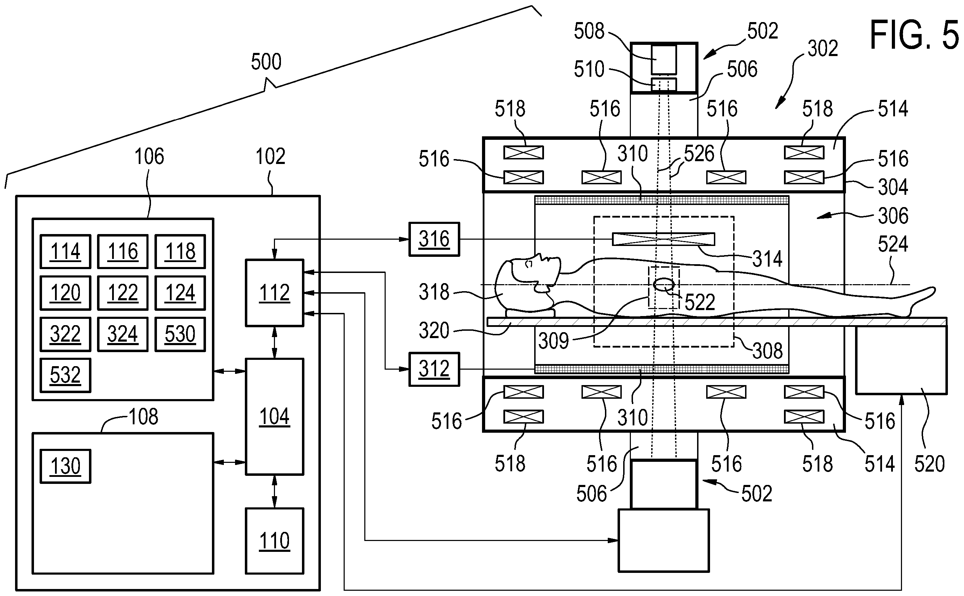

Fig. 5 shows a further example of a medical system 500. The medical system 500 of Fig. 5 is similar to the one shown in Fig. 3 except it additionally incorporates a radiotherapy unit 502 with the magnetic resonance imaging system 302.

The radiotherapy unit 502 comprises a gantry 506 and a radiotherapy source 508. The gantry 506 is for rotating the radiotherapy source 508 about an axis of gantry rotation 540. Adjacent to the radiotherapy source 508 is a collimator 510.

The magnet 304 shown in this embodiment is also standard cylindrical superconducting magnet. The magnet 304 has a cryostat 514 with superconducting coils within it 516. There are also superconducting shield coils 518 within the cryostat also.

The subject support 320 may be positioned by a mechanical positioning system 520. Within the subject 318 there is a target zone 522. The axis of gantry rotation 524 is coaxial in this particular embodiment with the cylindrical axis of the magnet 304. The subject support 320 has been positioned such that the target zone 522 lies on the axis 524 of gantry rotation. The radiation source 508 is shown as generating a radiation beam 526 which passes through the collimator 510 and through the target zone 522. As the radiation source 508 is rotated about the axis 524 the target zone 522 will always be targeted by the radiation beam 526. The radiation beam 526 passes through the cryostat 514 of the magnet. The magnetic field gradient coil may have also a gap which separate the magnetic field gradient coil into two sections. If present, this gap reduces attenuation of the radiation beam 526 by the magnetic field gradient coil 310. In some embodiments the radio frequency coil 314 may also have gaps or be separated to reduce attenuation of the radiation beam 526.

The radiation source may be constructed different technologies. In one example the radiation source 508 is a radioactive material which provides a beam of Gamma radiation. In another example the radiation source 508 is a X-ray source. In another example the radiation source is a source of charged particels. In other examples the radiation source is provided by a LINAC. It is also understood that the cylindical magnet is an example. A split coil magnet or an open magnet may also be used to provide a path of the radiation beam 526 to the subject 318.

The radiotherapy unit 102 and the mechanical positioning system 520 are additionally shown as being connected to a hardware interface 112 of the computer system 102.

The computer storage 106 is shown as additionally containing radiation therapy treatment data which is at least partly descriptive of the region of interest and contains a plan for irradiating the target zone 522. The computer storage 106 is further shown as containing radiotherapy control instructions 532 that have been generated using the radiation therapy treatment data 530 and the synthetic CT image 124.

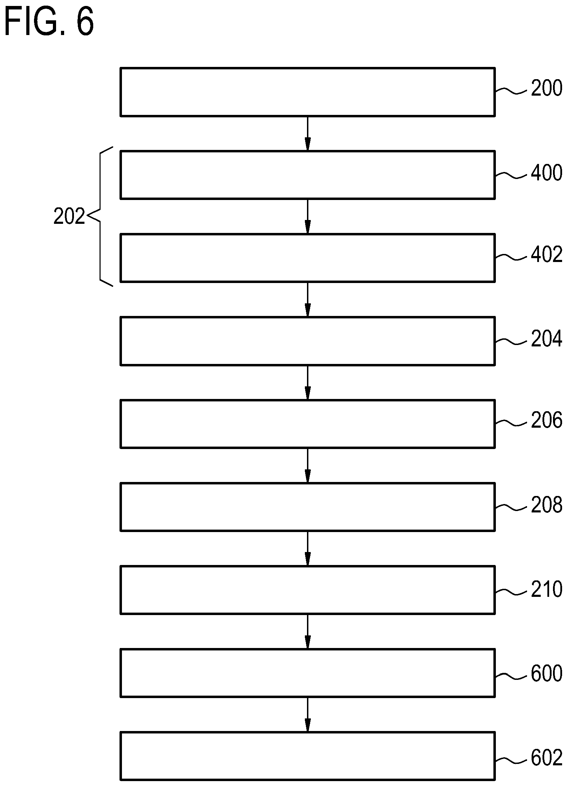

Fig. 6 shows a flowchart which illustrates a method of operating the medical system 500 of Fig. 5. The method shown in Fig. 6 is similar to that shown in Fig. 4 with several additional steps. After step 210 is performed radiation therapy treatment data 530 is received. The radiation therapy treatment data is at least partially descriptive of the region of interest. Next in step 602 the processor 104 calculates radiotherapy control instructions 532 for controlling the radiotherapy unit 502 using at least the synthetic CT image 124 and the radiation therapy treatment data 530.

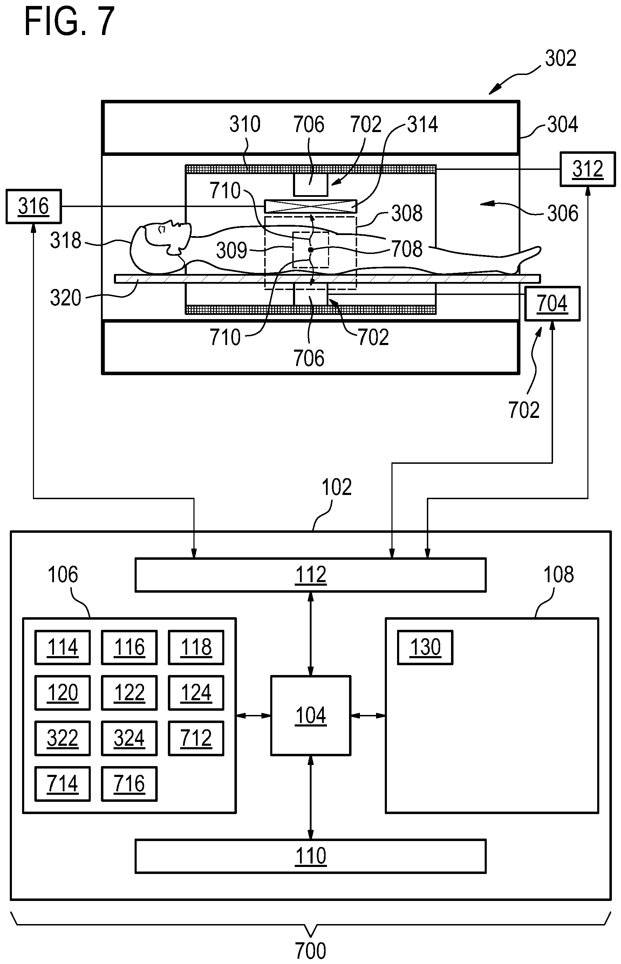

Fig. 7 shows a further example of a medical system 700. The medical system 700 shown in Fig. 7 is very similar to the medical system 300 shown in Fig. 3 except for the addition of an emission computer tomography system 702. The emission computer tomography system 702 comprises a control unit 704 and a detector ring 706. The detector ring 706 may contain detectors for detecting gamma radiation. The subject 318 may ingest or receive a radioisotope 708 which has a distribution within the body of the subject 318. In the case of positron emission tomography the radioisotope 708 emits a positron which then combines an electron and emits two gamma particles 710. These may then be detected by detectors in the detector ring 706. In single photon emission tomography a single gamma photon is emitted and detected by the detector ring 706. The machine-executable instructions 130 may then control the emission computer tomography system 702 to acquire gamma ray emission data 712 by recording data received by the detector ring 706. The gamma ray emission data 712 is shown as being stored in the computer storage 106. The computer storage 106 is further shown as containing a gamma ray absorption map 714 that is computed using the synthetic CT image 124. Finally the computer storage 106 is further shown as containing an emission computer tomography image 716 that was computed using the gamma ray absorption map 714 and the gamma ray emission data 712.

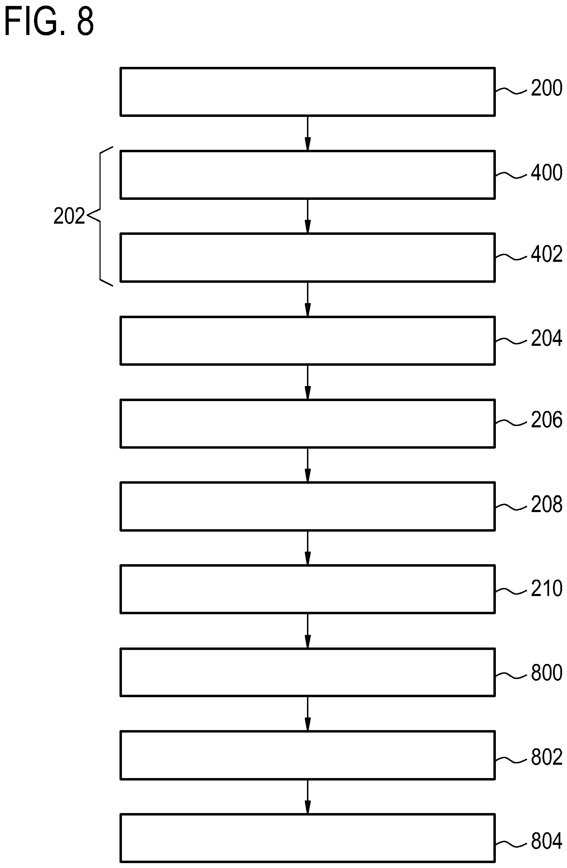

Fig. 8 shows a flowchart which illustrates a method of operating the medical system 700 of Fig. 7. The method shown in Fig. 8 is very similar to the method shown in Fig. 4 with the addition several additional steps. After step 210 is performed step 800 is performed. In step 800 the emission computer tomography system 702 is controlled to acquire the gamma ray emission data 712. Next in step 802 a gamma ray absorption map 714 is calculated using the synthetic CT image 124. Finally in step 804 the emission computer tomography image 716 is reconstructed using at least the gamma ray emission data 712 and the gamma ray absorption map 714.

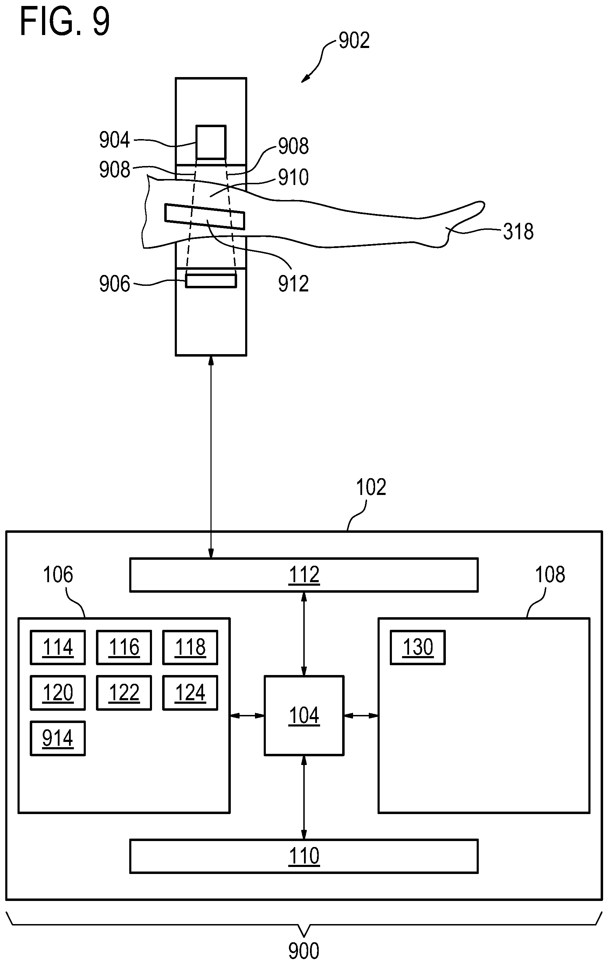

Fig. 9 shows a further example of a medical system 900. The medical system 900 is similar to that shown in Fig. 1. The medical system 900 additionally comprises an X-ray system 902. The features shown in Fig. 9 may be incorporated into any of the previous examples. That is to say the features in Fig. 9 may be incorporated into Fig. 1, Fig. 3, Fig. 7, and Fig. 5. The X-ray system 902 is for measuring the bone mineralization density data 114. The X-ray system 902 may for example be a dual energy X-ray absorptiometry unit or a quantitative computer tomography unit. In any case, the X-ray system 902 will comprise at least one X-ray source 904 and at least one X-ray detector 906. The X-ray source generates X-rays 908 which pass through a portion of a subject 318. The subject comprises soft tissue 910 and bone 912. The machine-executable instructions 130 may control the processor 104 which then in turn controls the X-ray system to acquire X-ray data 914. The X-ray data 914 can then be processed into the bone mineralization density data 114.

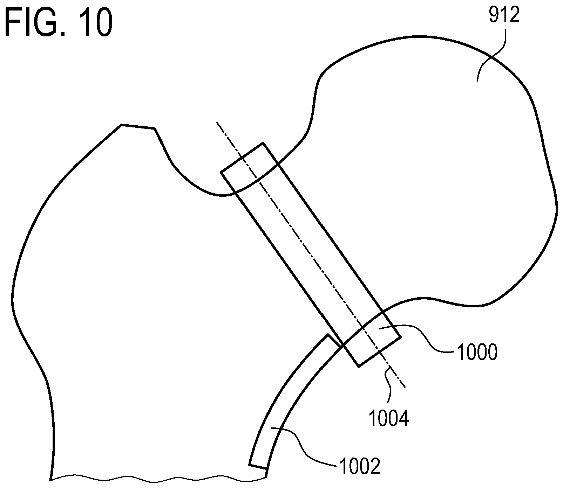

Fig. 10 is used to illustrate the different ways in which the bone mineralization density data 114 may be determined. Fig. 10 shows a section of a femoral bone 912. In a first case the X-ray system 902 is a dual energy X-ray absorptiometry unit. With this a conventional X-ray absorptiometry bone density measurement is made of the femoral bone by taking an X-ray image of the region of interest 1000. This region by itself is mostly comprised of spongy bone. The measurement for this area is then taken and then an empirical transfer function is applied to determine the dual energy X-ray absorptiometry bone density measurement value.

An alternative to this is also performed with a dual energy X-ray absorptiometry unit. In this case the image data is segmented and a different region of interest 1002 is taken. The region of interest 1002 comprises mostly cortical bone. The measurement from this portion of the image is then used to obtain a better estimate of the cortical bone density. A third alternative is that the X-ray system 902 is a quantitative computer tomography unit. The dashed line 1004 indicates a plane where a CT image of the femoral bone 912 is taken. From the CT image the spongy bone can be seen to be surrounded by harder cortical bone. Since the quantitative computer tomography unit is able to take quantitative measurements either the measured Hounsfield value can be converted into the Hounsfield value for the synthetic CT image or the measurement from the cortical bone can be first converted into a cortical bone density which is then further converted into the Hounsfield unit for the synthetic CT image.

CT-based radiation therapy plans (RTP) can correctly account for radiation attenuation in soft tissue and bones. MR-only based plans are increasingly used since superior in tumor and organ delineation. However, MR-based assessment of attenuation in bones remains a challenge since standard MRI does not yield any signal in the true bone compartment, but only signal from water and fat within trabecular bone. However, t there is a wide variation of especially the cortical bone mineralization and hence the respective attenuation.

Examples may provide a means of measuring bone mineralization density (BMD) for each RT patient and apply a transfer function to determine the HU value for bulk density assignment of the cortical bone compartment in MR-CAT. Hence, the BMD scan augments the MR-CAT approach.

The transfer function may be defined based on the acquisition of both, the BMD scan and a RTP-CT scan in a limited training patient cohort with age and sex matching the patient to be treated. Each CT scan may be evaluated to find the mean HU value for bulk density assignment of the cortical bone part as in MR-CAT. The transfer function is defined as a parametrized function that translates the BDM value to this bulk HU value.

BMD may be measured with conventional BMD equipment and algorithms as used widely for managing of osteoporosis patients in the most simple embodiment. Several dedicated evaluation approaches for the BDM scan are proposed as additional embodiments to improve selectivity of BDM for the cortical bone compartment.

Attenuation maps for use in radiation therapy planning (RTP) are conventionally derived from computed tomography (CT) scans. MRI is clearly superior to CT in the delineation of tumor and risk organs. However, so far MRI has not been utilized much for standalone radiation therapy simulation since MR-based assessment of attenuation maps is a difficult task, especially in trabecular and even more cortical bone. Several approaches to simulate CT images and derive respective attenuation maps for MR-only based RTP have been reported in literature:
One approach is to use an atlas-based method. In this approach, a population-average density map (the atlas) is constructed by registering a cohort of CT images non-rigidly onto each other and combining those. This average density map is then mapped to a particular patient by a non-rigid registration.

### Bulk density assignment

This approach first uses one or more different MRI contrasts to categorize into few major tissue classes (e.g. air, fat, water-rich tissue, trabecular bone, and cortical bone). In the next step, each voxel is assigned a density value (pseudo-HU value) based on a combination of average population values and literature values of HU values.

### Model-based voxel-wise density assignment

This approach establishes a conversion model between one or more MR contrasts and a simulated HU value. The model is trained on a limited number of co-registered CT and MR datasets. An example of this technique uses a Gaussian mixture regression model to link the CT contrast to five different MR contrasts obtained from three different MR scans: one T2 weighted 3D spin echo based sequence and two dual echo ultrashort echo time MRI sequences with different echo times and flip angles. A further example treats bone and the other tissues in separate models.

### Bone mineralization density assessment (BMD)

In the following, a brief introduction on bone density assessments is given. Bone mineral density (BMD) is a measurement at organ level and is performed regularly in clinical care, e.g. in osteoporosis patients. BMD is measured by mainly two methods: Dual energy X-ray absorptiometry (DXA) to measure areal BMD and quantitative CT (QCT) to measure volumetric BMD. Both methods inherently provide a global evaluation of bone mineralization, as they do not separate pure bone-tissue mineral from porosity (haversian canals and bone marrow spaces).

In DXA, bone mineral content (measured as the total attenuation of the X-ray by the bones being scanned) is divided by the area of the bone as projected onto the X-ray image. Hence, it is not an accurate measurement of true bone mineral density, which is mass divided by a volume. QCT is capable of measuring the bone's volume, and is, therefore, not susceptible to the confounding effect of bone-size in the way that DXA results are. Still, DXA provides a fairly accurate measure of bone mineral content in clinical practice because there are standards for patient positioning and fully-automatic algorithms to define the ROI e.g. in the femoral neck and evaluate the BDM. To avoid an overestimation of bone mineral deficits, DXA scores may be compared to reference data for the same gender and age (by calculating a Z-score = signed number of SDs from mean).

The U.S. Preventive Services Task Force recommends that women over the age of 65 should get a DXA scan for osteoporosis screening and prevention of associated bone fractures. The date at which men should be tested is uncertain but some sources recommend age 70. The cost of a DXA-BMD measurement is low (42 EUR in Germany). It lasts for about 15-30min and involves only 1-2µS of radiation. In comparison, a transatlantic flight is associated with a dose of 50µS.

QCT can be performed on standard CT scanners as well as dedicated peripheral (pQCT) scanners. QCT measurements of the spine and the hip are performed on clinical all-purpose, total body CT scanners equipped with special analysis software. pQCT scanners have been specifically developed for the quantitative determination of BMD in the forearm and the tibia. They are less expensive, more mobile, and involve much less radiation (3 µSv per slice) than whole body clinical CT scanners for non-peripheral BDM (0.2-1.5 mSv)

### Assessment of bone mineral density distribution

In difference to BMD, the bone mineral density distribution (BMDD) can be measured at a microscopic level using small bone samples obtained by bone biopsy and yields a histogram of the calcium weight % in the sample. Mostly, mean and width of this distribution are used to compare different patients. The qBEI method may be used to measure BMDD and may for example have a resolution of resolution 2x2x2µm³ and may have an inter-assay variance of 0.3% C.V. (coefficient of variance) for the mean Calcium-content.

### BMD data in healthy and diseased individuals

BMD may varying considerably between individuals (SD of is approx. 20% of mean) and depends on age, sex, health state and other parameters.

### BMDD in in healthy and diseased individuals

BMDD in trabecular bone of different skeletal sites (transiliac bone, vertebrae, femoral neck and head, patella) from adult individuals (age 25-90 years), of different ethnicity (African and Caucasian Americans) and gender analyzed in healthy individuals shows a remarkably small biological variance (mean Ca weight % = 22.2 ± 0.45 results in a 2% relative variation) suggesting the existence of an evolutionary optimum with respect to its biomechanical performance.

While the MR-based assignment of HU works relatively well in soft tissues, all three approaches described above suffer from the difficulty to assign meaningful HU in bone tissue. The CT-atlas-based approach assigns the same normalized HUs to all patients based on those derived from CT-atlas. In addition, it suffers from registration errors.

The bulk density assignment assigns one single HU to all voxels identified as trabecular bone and a second HU value to all voxels identified as cortical bone. These two values are derived from average population values and literature values. In addition, this method suffers from segmentation inaccuracy leading to incorrect tissue assignments.

The approaches for model-based voxel-wise density assignment suffer from the difficulty of finding a transfer function from MR intensities to HU values especially for cortical bone. In addition, the transfer functions derived from a limited patient cohort is applied to all patients.

In essence, all three approaches do not take into account any inter-patient variations of bone density and respective HU values. However, those variations are much larger than in soft tissue, especially in the cortical bone.

Examples may provide for a means to measure bone mineralization density (BMD) for each radiotherapy (RT) patient and apply a transfer function to determine the HU value for bulk density assignment of the cortical bone compartment in MR-CAT. Hence, the BMD scan augments the MR-CAT approach.

The transfer function may be defined based on the acquisition of both, the BMD scan and a RTP-CT scan in a limited training patient cohort with age and sex matching the patient to be treated. Each CT scan is evaluated to find the mean HU value for bulk density assignment of the cortical bone part as in MR-CAT. The transfer function may be defined as a parametrized function that translates the BDM value to this bulk HU value.

An example method may contain one or more of the following steps:
1. Acquire DXA scans and conventional CT scans for RTP in a training cohort of patients that is representative of actual patients (age, sex, weight, size).
2. Segment cortical bone in CT scans with state-of-the-art methods, potentially manually. Preferably segment those parts that are most relevant for attenuation, e.g. the hip and sacrum in prostate patients. Calculate the HU value for bulk density assignment for cortical bone as in MRCAT.
3. Define a transfer function that describes the translation from the DXA BMD value to the HU value used for bulk assignment in cortical bone.
4. For future patients (beyond the "training" patients), acquire only the DXA scan and the MRIs required for MRCAT, but not the CT scan.
5. Use the DXA value and the trained transfer function to determine the HU value for bulk density assignment in cortical bone.

The use of conventional DXA may have the drawback that conventional DXA does not distinguish cortical from trabecular bone, and reports total BMD for both types as evaluated in the neck of the femoral bone (femoral neck) as one value with unit g/cm². The bulk mass of cortical bone in the top part of the femoral bone is known to be located on the inferior side and it thickens from the femoral on neck further inferior.

As an alternative the BEM may be evaluated such that the BDM only for a ROI in that area as depicted in region 1002 in Fig. 10, which more closely reflects the BDM of cortical bone. This cortical ROI should be found as a layer of defined width and length adjacent to the neck ROI delineated as green rectangle. The width of the cortical ROI may be defined as e.g. 5% percent of the width of the minimum width of the neck. The length of the cortical ROI may be defined as e.g. 50% of the width of the minimum width of the neck. The width may be defined as to increase linearly from a smaller upper width to a larger lower width

DXA can distinguish different tissue compartments and hence evaluate the density of bone separately, but it is a projection technique that reports average BDM in certain ROIs in g/cm². This limitation is overcome by pQCT which measures BDM in g/cm³ and can resolve the volume in 3D. Hence, cortical and trabecular bone compartments can be assessed separately.

In QCT, BMD is determined from the measured CT parameters. This step is based on a linear calibration of CT number to BMD using a phantom with bone equivalent calibration materials of density. This step may be omitted since it more simple to directly relate the HU values measured in QCT to the HU values measured in the standard CT. So, in embodiment 3 pQCT e.g. acquired in the femoral bone is used instead of DXA in step 1. Cortical bone is segmented and the mean HU for this segment is assessed. The remaining steps 2 to 5 are performed in analogy to embodiment 1. Hence, also in step 4, DXA is replaced by pQCT.

The invention is defined in the following claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: medical system
- 102: computer
- 104: processor
- 106: computer storage
- 108: computer memory
- 110: user interface
- 112: hardware interface
- 114: bone mineralization density data
- 116: magnetic resonance image
- 118: image segmentation
- 120: cortical bone Hounsfield value
- 122: Hounsfield unit mapping
- 124: synthetic CT image
- 130: machine executable instructions
- 200: receive bone mineralization density data
- 202: receive a magnetic resonance image of a region of interest
- 204: calculate an image segmentation by segmenting the magnetic resonance image into a set of tissue types
- 206: calculate a cortical bone Hounsfield value using the bone mineralization density data
- 208: calculate a Hounsfield unit mapping by assigning at least one Hounsfield unit value to each of the set of tissue types in the image segmentation
- 210: calculate the synthetic CT image using the Hounsfield unit mapping
- 300: medical system
- 302: magnetic resonance imaging system
- 304: magnet
- 306: bore of magnet
- 308: measurement zone or imaging zone
- 309: region of interest
- 310: magnetic field gradient coils
- 312: magnetic field gradient coil power supply
- 314: radio-frequency coil
- 316: transceiver
- 318: subject
- 320: subject support
- 322: pulse sequence commands
- 324: magnetic resonance data
- 400: controlling the magnetic resonance imaging system to acquire magnetic resonance data from at least the region of interest
- 402: reconstructing the magnetic resonance image from the magnetic resonance imaging system
- 500: medical system
- 502: radiotherapy unit
- 506: gantry
- 508: radiotherapy source
- 510: collimator
- 514: cryostat
- 516: superconducting coil
- 518: superconducting shield coil
- 520: mechanical positioning system
- 522: target zone
- 524: axis of gantry rotation
- 526: radiation beam
- 530: radiation therapy treatment data
- 532: radiation therapy control instructions
- 600: receive radiation therapy treatment data that is at least partially descriptive of the region of interest
- 602: calculate radiotherapy control instructions for controlling the radiotherapy unit using at least the synthetic CT image and the radiotherapy treatment data
- 700: medical system
- 702: emission computed tomography system
- 704: control unit
- 706: detector ring
- 708: radio isotope
- 710: gamma radiation
- 712: gamma ray emission data
- 714: gamma ray absorption map
- 716: emission computed tomography image
- 800: control the emission computed tomography system to acquire gamma ray emission data
- 802: calculate a gamma ray absorption map using the synthetic CT image
- 804: reconstruct an emission computed tomography image using at least the gamma ray emission data and the gamma ray absorption map
- 900: medical system
- 902: X-ray system
- 904: X-ray source
- 906: X-ray detector
- 908: X-rays
- 910: soft tissue
- 912: bone
- 914: X-ray data
- 1000: region of interest
- 1002: region of interest
- 1004: plane of CT scan

## Claims

1. A computer program product for calculating a synthetic CT image (124), wherein the computer program product comprises machine executable instructions (130) for execution by a processor (104), wherein execution of the machine executable instructions causes the processor to:
- receive (200) bone mineralization density data (114);
- receive (202) a magnetic resonance image (116) of a region of interest (309);
- calculate (204) an image segmentation (118) by segmenting the magnetic resonance image into a set of tissue types, wherein the set of tissue types comprises a cortical bone segmentation;
- calculate (206) a cortical bone Hounsfield value (120) using the bone mineralization density data;
- calculate (208) a Hounsfield unit mapping (122) by assigning at least one Hounsfield unit value to each of the set of tissue types in the image segmentation, wherein the cortical bone segmentation is assigned the cortical bone Hounsfield value; and
- calculate (210) the synthetic CT image using the Hounsfield unit mapping.

2. The computer program product of claim 1, wherein execution of the machine executable instructions further cause the processor to:
- receive (600) radiation therapy treatment data (530) that is at least partially descriptive of the region of interest; and
- calculate (602) radiotherapy control instructions (532) using at least the synthetic CT image and the radiotherapy treatment data.

3. The computer program product of claim 1 or 2, wherein the bone mineralization density data comprises dual energy X-ray absorptiometry data.

4. The computer program product of claim 3, wherein calculating the cortical bone Hounsfield value comprises determining a dual energy X-ray absorptiometry bone density measurement value from the dual energy X-ray absorptiometry data, wherein the cortical bone Hounsfield value is determined by comparing applying an empirical transfer function to the dual energy X-ray absorptiometry bone density measurement value.

5. The computer program product of claim 3, wherein the dual energy X-ray absorptiometry data comprises a dual energy X-ray absorptiometry image, wherein calculating the cortical bone Hounsfield value comprises;
- locating a cortical bone region (1002) in the dual energy X-ray absorptiometry image by segmenting the dual energy X-ray absorptiometry image;
- determining a cortical bone density from the cortical bone region; and
- assigning the cortical bone Hounsfield value using the cortical bone density and a look up table.

6. The computer program product of claim 1 or 2, wherein the bone mineralization density data comprises a quantitative computed tomography image, wherein execution of the machine executable instructions further cause the processor to locate a cortical bone region in the quantitative computed tomography image by segmenting the quantitative computed tomography image, wherein execution of the machine executable instructions further cause the processor to assign the cortical bone Hounsfield value using the cortical bone region in the quantitative computed tomography image.

7. A medical imaging method, wherein the method comprises:
- receiving (200) bone mineralization density data (114);
- receiving (202) a magnetic resonance image (116) of a region of interest (309);
- calculating (204) an image segmentation by segmenting the magnetic resonance image into a set of tissue types, wherein the set of tissue types comprises a cortical bone segmentation;
- calculating (206) a cortical bone Hounsfield value (120) using the bone mineralization density data;
- calculating (208) a Hounsfield unit mapping (122) by assigning at least one Hounsfield unit value to each of the set of tissue types in the image segmentation, wherein the cortical bone segmentation is assigned the cortical bone Hounsfield value; and
- calculating (210) a synthetic CT image (124) using the Hounsfield unit mapping.

8. A medical system (100, 300, 500, 700, 900), wherein the medical system comprises:
- a memory (108) for storing machine executable instructions (130);
- a processor (104) for controlling the medical system, wherein execution of the machine executable instructions causes the processor to:
• receive (200) bone mineralization density data (114);
• receive (202) a magnetic resonance image (116) of a region of interest (309);
• calculate (204) an image segmentation by segmenting the magnetic resonance image into a set of tissue types, wherein the set of tissue types comprises a cortical bone segmentation;
• calculate (206) a cortical bone Hounsfield value (120) using the bone mineralization density data;
• calculate (208) a Hounsfield unit mapping (122) by assigning at least one Hounsfield unit value to each of the set of tissue types in the image segmentation, wherein the cortical bone segmentation is assigned the cortical bone Hounsfield value; and
• calculate (210) the synthetic CT image using the Hounsfield unit mapping.

9. The medical system of claim 13, wherein the medical system further comprises a magnetic resonance imaging system (302), wherein execution of the machine executable instructions further cause the processor to receive a magnetic resonance image of a region of interest by:
• controlling (400) the magnetic resonance imaging system to acquire magnetic resonance data from at least the region of interest; and
• reconstructing (402) the magnetic resonance image from the magnetic resonance imaging system.

10. The medical system of claim 8 or 9, wherein the medical system comprises a radiotherapy unit (502), wherein execution of the machine executable instructions further cause the processor to:
- receive (600) radiation therapy treatment data (530) that is at least partially descriptive of the region of interest; and
- calculate (602) radiotherapy control instructions (532) for controlling the radiotherapy unit using at least the synthetic CT image and the radiotherapy treatment data.

11. The medical system of claim 8, 9, or 10, wherein the medical system further comprises an emission computed tomography system (702), wherein execution of the machine executable instructions further causes the processor to:
- control (800) the emission computed tomography system to acquire gamma ray emission data (712),
- calculate (802) a gamma ray absorption map (714) using the synthetic CT image, and
- reconstruct (804) an emission computed tomography image (716) using at least the gamma ray emission data and the gamma ray absorption map.

12. The medical system of any one of claims 8 through 11, wherein the medical system further comprises a dual energy X-ray absorptiometry unit (902), wherein the radiation therapy treatment data comprises a dual energy X-ray absorptiometry image (914), and wherein the processor receives the bone mineralization density data from the dual energy X-ray absorptiometry unit.

13. The medical system of claim 12, wherein calculating the cortical bone Hounsfield value comprises determining a dual energy X-ray absorptiometry bone density measurement value from the dual energy X-ray absorptiometry image, wherein the cortical bone Hounsfield value is determined by applying an empirical transfer function to the dual energy X-ray absorptiometry bone density measurement value.

14. The medical system of claim 12, wherein calculating the cortical bone Hounsfield value comprises;
- locating a cortical bone region in the dual energy X-ray absorptiometry image by segmenting the dual energy X-ray absorptiometry image;
- determining a cortical bone density from the cortical bone region; and
- assigning the cortical bone Hounsfield value using the cortical bone density and a look up table.

15. The medical system of any one of claims 8 through 11, wherein the medical system further comprises a quantitative computed tomography unit (902), wherein the processor receives the bone mineralization density data from the quantitative computed tomography unit, wherein the bone mineralization density data comprises a quantitative computed tomography image (914), wherein execution of the machine executable instructions causes the processor to locate a cortical bone region in the quantitative computed tomography image by segmenting the quantitative computed tomography image, wherein execution of the machine executable instructions causes the processor to assign the cortical bone Hounsfield value using the cortical bone region in the quantitative computed tomography image.

## Patentansprüche

1. Computerprogrammprodukt zum Berechnen eines synthetischen CT-Bildes (124), wobei das Computerprogrammprodukt maschinenausführbare Anweisungen (130) zur Ausführung durch einen Prozessor (104) umfasst, wobei die Ausführung der maschinenausführbaren Anweisungen bewirkt, dass der Prozessor:
- Knochenmineralisierungsdichtedaten (114) empfängt (200);
- ein Magnetresonanzbild (116) eines Bereichs von Interesse (309) empfängt (202);
- durch Segmentieren des Magnetresonanzbildes in eine Reihe von Gewebetypen eine Bildsegmentierung (118) berechnet (204), wobei die Reihe von Gewebetypen eine Segmentierung von kortikalem Knochen umfasst;
- unter Verwendung der Knochenmineralisierungsdichtedaten einen Hounsfield-Wert von kortikalem Knochen (120) berechnet (206);
- durch Zuordnen mindestens eines Hounsfield-Einheit-Wertes zu jedem der Reihe von Gewebetypen in der Bildsegmentierung eine Hounsfield-Einheit-Kartierung (122) berechnet (208), wobei die Segmentierung von kortikalem Knochen dem Hounsfield-Wert von kortikalem Knochen zugeordnet wird; und
- unter Verwendung der Hounsfield-Einheit-Kartierung das synthetische CT-Bild berechnet (210).

2. Computerprogrammprodukt nach Anspruch 1, wobei die Ausführung der maschinenausführbaren Anweisungen bewirkt, dass der Prozessor:
- Strahlentherapiebehandlungsdaten (530), die mindestens teilweise den Bereich von Interesse beschreiben, empfängt (600); und
- unter Verwendung mindestens des synthetischen CT-Bildes und der Strahlentherapiebehandlungsdaten Strahlentherapiesteuerungsanweisungen (532) berechnet (602).

3. Computerprogrammprodukt nach Anspruch 1 oder 2, wobei die Knochenmineralisierungsdichtedaten Dual-Energy-Röntgenabsorptiometrie-Daten umfassen.

4. Computerprogrammprodukt nach Anspruch 3, wobei das Berechnen des Hounsfield-Wertes von kortikalem Knochen das Bestimmen eines Dual-Energy-Röntgenabsorptiometrie-Knochendichtemessungswertes aus den Dual-Energy-Röntgenabsorptiometrie-Daten umfasst, wobei der Hounsfield-Wert von kortikalem Knochen durch Vergleichen der Anwendung einer empirischen Transferfunktion auf den Dual-Energy-Röntgenabsorptiometrie-Knochendichtemessungswert bestimmt wird.

5. Computerprogrammprodukt nach Anspruch 3, wobei die Dual-Energy-Röntgenabsorptiometrie-Daten ein Dual-Energy-Röntgenabsorptiometrie-Bild umfassen, wobei das Berechnen des Hounsfield-Wertes von kortikalem Knochen Folgendes umfasst:
- Lokalisieren eines Bereichs von kortikalem Knochen (1002) in dem Dual-Energy-Röntgenabsorptiometrie-Bild durch Segmentieren des Dual-Energy-Röntgenabsorptiometrie-Bildes;
- Bestimmen einer Dichte von kortikalem Knochen aus dem Bereich von kortikalem Knochen; und
- Zuordnen des Hounsfield-Wertes von kortikalem Knochen unter Verwendung der Dichte von kortikalem Knochen und einer Nachschlagetabelle.

6. Computerprogrammprodukt nach Anspruch 1 oder 2, wobei die Knochenmineralisierungsdichtedaten ein quantitatives Computertomografie-Bild umfassen, wobei die Ausführung der maschinenausführbaren Anweisungen weiter bewirkt, dass der Prozessor einen Bereich von kortikalem Knochen in dem quantitativen Computertomografie-Bild durch Segmentieren des quantitativen Computertomografie-Bildes lokalisiert, wobei die Ausführung der maschinenausführbaren Anweisungen weiter bewirkt, dass der Prozessor den Hounsfield-Wert von kortikalem Knochen unter Verwendung des Bereichs von kortikalem Knochen in dem quantitativen Computertomografie-Bild zuordnet.

7. Medizinisches Bildgebungsverfahren, wobei das Verfahren Folgendes umfasst:
- Empfangen (200) von Knochenmineralisierungsdichtedaten (114);
- Empfangen (202) eines Magnetresonanzbildes (116) eines Bereichs von Interesse (309);
- Berechnen (204) einer Bildsegmentierung durch Segmentieren des Magnetresonanzbildes in eine Reihe von Gewebetypen, wobei die Reihe von Gewebetypen eine Segmentierung von kortikalem Knochen umfasst;
- Berechnen (206) eines Hounsfield-Wertes von kortikalem Knochen (120) unter Verwendung der Knochenmineralisierungsdichtedaten;
- Berechnen (208) einer Hounsfield-Einheit-Kartierung (122) durch Zuordnen mindestens eines Hounsfield-Einheit-Wertes zu jedem der Reihe von Gewebetypen in der Bildsegmentierung, wobei die Segmentierung von kortikalem Knochen dem Hounsfield-Wert von kortikalem Knochen zugeordnet wird; und
- Berechnen (210) eines synthetischen CT-Bildes (124) unter Verwendung der Hounsfield-Einheit-Kartierung.

8. Medizinisches System (100, 300, 500, 700, 900), wobei das medizinische System Folgendes umfasst:
- einen Speicher (108) zum Speichern maschinenausführbarer Anweisungen (130);
- einen Prozessor (104) zum Steuern des medizinischen Systems, wobei die Ausführung der maschinenausführbaren Anweisungen bewirkt, dass der Prozessor:
• Knochenmineralisierungsdichtedaten (114) empfängt (200);
• ein Magnetresonanzbild (116) eines Bereichs von Interesse (309) empfängt (202);
• durch Segmentieren des Magnetresonanzbildes in eine Reihe von Gewebetypen eine Bildsegmentierung berechnet (204), wobei die Reihe von Gewebetypen eine Segmentierung von kortikalem Knochen umfasst;
• unter Verwendung der Knochenmineralisierungsdichtedaten einen Hounsfield-Wert von kortikalem Knochen (120) berechnet (206);
• durch Zuordnen mindestens eines Hounsfield-Einheit-Wertes zu jedem der Reihe von Gewebetypen in der Bildsegmentierung eine Hounsfield-Einheit-Kartierung (122) berechnet (208), wobei die Segmentierung von kortikalem Knochen dem Hounsfield-Wert von kortikalem Knochen zugeordnet wird; und
• unter Verwendung der Hounsfield-Einheit-Kartierung das synthetische CT-Bild berechnet (210).

9. Medizinisches System nach Anspruch 13, wobei das medizinische System weiter ein Magnetresonanzbildgebungssystem (302) umfasst, wobei die Ausführung der maschinenausführbaren Anweisungen weiter bewirkt, dass der Prozessor ein Magnetresonanzbild eines Bereichs von Interesse empfängt durch:
• Steuern (400) des Magnetresonanzbildgebungssystems, um Magnetresonanzdaten von mindestens dem Bereich von Interesse zu erfassen; und
• Rekonstruieren (402) des Magnetresonanzbildes von dem Magnetresonanzbildgebungssystem.
•

10. Medizinisches System nach Anspruch 8 oder 9, wobei das medizinische System eine Radiotherapieeinheit (502) umfasst, wobei die Ausführung der maschinenausführbaren Anweisungen weiter bewirkt, dass der Prozessor:
- Strahlentherapiebehandlungsdaten (530) empfängt (600), die mindestens teilweise den Bereich von Interesse beschreiben; und
- Radiotherapiesteuerungsanweisungen (532) zum Steuern der Radiotherapieeinheit unter Verwendung mindestens des synthetischen CT-Bildes und der Radiotherapiebehandlungsdaten berechnet (602).

11. Medizinisches System nach Anspruch 8, 9 oder 10, wobei das medizinische System weiter ein Emissionscomputertomografiesystem (702) umfasst, wobei die Ausführung der maschinenausführbaren Anweisungen weiter bewirkt, dass der Prozessor:
- das Emissionscomputertomografiesystem steuert (800), um Gammastrahlemissionsdaten (712) zu erfassen,
- unter Verwendung des synthetischen CT-Bildes eine Gammastrahlabsorptionskarte (714) berechnet (802) und
- unter Verwendung mindestens der Gammastrahlemissionsdaten und der Gammastrahlabsorptionskarte ein Emissionscomputertomografiebild (716) rekonstruiert (804).

12. Medizinisches System nach einem der Ansprüche 8 bis 11, wobei das medizinische System weiter eine Dual-Energy-Röntgenabsorptiometrie-Einheit (902) umfasst, wobei die Strahlentherapiebehandlungsdaten ein Dual-Energy-Röntgenabsorptiometrie-Bild (914) umfassen und wobei der Prozessor die Knochenmineralisierungsdichtedaten von der Dual-Energy-Röntgenabsorptiometrie-Einheit empfängt.

13. Medizinisches System nach Anspruch 12, wobei das Berechnen des Hounsfield-Wertes von kortikalem Knochen das Bestimmen eines Dual-Energy-Röntgenabsorptiometrie-Knochendichtemessungswertes aus dem Dual-Energy-Röntgenabsorptiometrie-Bild umfasst, wobei der Hounsfield-Wert von kortikalem Knochen durch Anwenden einer empirischen Transferfunktion auf den Dual-Energy-Röntgenabsorptiometrie-Knochendichtemessungswert bestimmt wird.

14. Medizinisches System nach Anspruch 12, wobei das Berechnen des Hounsfield-Wertes von kortikalem Knochen Folgendes umfasst:
- Lokalisieren eines Bereichs von kortikalem Knochen in dem Dual-Energy-Röntgenabsorptiometrie-Bild durch Segmentieren des Dual-Energy-Röntgenabsorptiometrie-Bildes;
- Bestimmen einer Dichte von kortikalem Knochen aus dem Bereich von kortikalem Knochen; und
- Zuordnen des Hounsfield-Wertes von kortikalem Knochen unter Verwendung der Dichte von kortikalem Knochen und einer Nachschlagetabelle.

15. Medizinisches System nach einem der Ansprüche 8 bis 11, wobei das medizinische System weiter eine quantitative Computertomografie-Einheit (902) umfasst, wobei der Prozessor die Knochenmineralisierungsdichtedaten von der quantitativen Computertomografie-Einheit empfängt, wobei die Knochenmineralisierungsdichtedaten ein quantitatives Computertomografie-Bild (914) umfassen, wobei die Ausführung der maschinenausführbaren Anweisungen bewirkt, dass der Prozessor einen Bereich von kortikalem Knochen in dem quantitativen Computertomografie-Bild durch Segmentieren des quantitativen Computertomografie-Bildes lokalisiert, wobei die Ausführung der maschinenausführbaren Anweisungen bewirkt, dass der Prozessor den Hounsfield-Wert von kortikalem Knochen unter Verwendung des Bereichs von kortikalem Knochen in dem quantitativen Computertomografie-Bild zuordnet.

## Revendications

1. Produit de programme informatique pour calculer une image TDM synthétique (124), dans lequel le produit de programme informatique comprend des instructions exécutables par machine (130) pour exécution par un processeur (104), dans lequel l'exécution des instructions exécutables par machine conduit le processeur à :
- recevoir (200) des données de densité de minéralisation osseuse (114) ;
- recevoir (202) une image de résonance magnétique (116) d'une région d'intérêt (309) ;
- calculer (204) une segmentation d'image (118) par segmentation de l'image de résonance magnétique en un ensemble de types de tissu, dans lequel l'ensemble de types de tissu comprend une segmentation d'os cortical ;
- calculer (206) une valeur Hounsfield d'os cortical (120) en utilisant les données de densité de minéralisation osseuse ;
- calculer (208) une cartographie d'unité Hounsfield (122) par attribution d'au moins une valeur d'unité Hounsfield à chacun de l'ensemble de types de tissu dans la segmentation d'image, dans lequel la segmentation d'os cortical se voit attribuée la valeur Hounsfield d'os cortical ; et
- calculer (210) l'image TDM synthétique en utilisant la cartographie d'unité Hounsfield.

2. Produit de programme informatique selon la revendication 1, dans lequel l'exécution des instructions exécutables par machine conduit en outre le processeur à :
- recevoir (600) des données de traitement de radiothérapie (530) qui décrivent au moins partiellement la région d'intérêt ; et
- calculer (602) des instructions de commande de radiothérapie (532) en utilisant au moins l'image TDM synthétique et les données de traitement de radiothérapie.

3. Produit de programme informatique selon la revendication 1 ou 2, dans lequel les données de densité de minéralisation osseuse comprennent des données d'absorptiométrie biénergétique à rayons X.

4. Produit de programme informatique selon la revendication 3, dans lequel le calcul de la valeur Hounsfield d'os cortical comprend la détermination d'une valeur de mesure de densité osseuse par absorptiométrie biénergétique à rayons X à partir des données d'absorptiométrie biénergétique à rayons X, dans lequel la valeur Hounsfield d'os cortical est déterminée par comparaison de l'application d'une fonction de transfert empirique à la valeur de mesure de densité osseuse par absorptiométrie biénergétique à rayons X.

5. Produit de programme informatique selon la revendication 3, dans lequel les données d'absorptiométrie biénergétique à rayons X comprennent une image d'absorptiométrie biénergétique à rayons X, dans lequel le calcul de la valeur Hounsfield d'os cortical comprend ;
- la localisation d'une région d'os cortical (1002) dans l'image d'absorptiométrie biénergétique à rayons X par segmentation de l'image d'absorptiométrie biénergétique à rayons X ;
- la détermination d'une densité d'os cortical à partir de la région d'os cortical ; et
- l'attribution de la valeur Hounsfield d'os cortical en utilisant la densité d'os cortical et une table de conversion.

6. Produit de programme informatique selon la revendication 1 ou 2, dans lequel les données de densité de minéralisation osseuse comprennent une image de tomodensitométrie quantitative, dans lequel l'exécution des instructions exécutables par machine conduit en outre le processeur à localiser une région d'os cortical dans l'image de tomodensitométrie quantitative par segmentation de l'image de tomodensitométrie quantitative, dans lequel l'exécution des instructions exécutables par machine conduit en outre le processeur à attribuer la valeur Hounsfield d'os cortical en utilisant la région d'os cortical dans l'image de tomodensitométrie quantitative.

7. Procédé d'imagerie médicale, dans lequel le procédé comprend :
- la réception (200) de données de densité de minéralisation osseuse (114) ;
- la réception (202) d'une image de résonance magnétique (116) d'une région d'intérêt (309) ;
- le calcul (204) d'une segmentation d'image par segmentation de l'image de résonance magnétique en un ensemble de types de tissu, dans lequel l'ensemble de types de tissu comprend une segmentation d'os cortical ;
- le calcul (206) d'une valeur Hounsfield d'os cortical (120) en utilisant les données de densité de minéralisation osseuse ;
- le calcul (208) d'une cartographie d'unité Hounsfield (122) par attribution d'au moins une valeur d'unité Hounsfield à chacun de l'ensemble de types de tissu dans la segmentation d'image, dans lequel la segmentation d'os cortical se voit attribuée la valeur Hounsfield d'os cortical ; et
- le calcul (210) d'une image TDM synthétique (124) en utilisant la cartographie d'unité Hounsfield.

8. Système médical (100, 300, 500, 700, 900), dans lequel le système médical comprend :
- une mémoire (108) pour stocker des instructions exécutables par machine (130) ;
- un processeur (104) pour commander le système médical, dans lequel l'exécution des instructions exécutables par machine conduit le processeur à :
• recevoir (200) des données de densité de minéralisation osseuse (114) ;
• recevoir (202) une image de résonance magnétique (116) d'une région d'intérêt (309) ;
• calculer (204) une segmentation d'image par segmentation de l'image de résonance magnétique en un ensemble de types de tissu, dans lequel l'ensemble de types de tissu comprend une segmentation d'os cortical ;
• calculer (206) une valeur Hounsfield d'os cortical (120) en utilisant les données de densité de minéralisation osseuse ;
• calculer (208) une cartographie d'unité Hounsfield (122) par attribution d'au moins une valeur d'unité Hounsfield à chacun de l'ensemble de types de tissu dans la segmentation d'image, dans lequel la segmentation d'os cortical se voit attribuée la valeur Hounsfield d'os cortical ; et
• calculer (210) l'image TDM synthétique en utilisant la cartographie d'unité Hounsfield.

9. Système médical selon la revendication 13, dans lequel le système médical comprend en outre un système d'imagerie par résonance magnétique (302), dans lequel l'exécution des instructions exécutables par machine conduit en outre le processeur à recevoir une image de résonance magnétique d'une région d'intérêt par :
• commande (400) du système d'imagerie par résonance magnétique pour acquérir des données de résonance magnétique à partir d'au moins la région d'intérêt ; et
• reconstruction (402) de l'image de résonance magnétique à partir du système d'imagerie par résonance magnétique.

10. Système médical selon la revendication 8 ou 9, dans lequel le système médical comprend une unité de radiothérapie (502), dans lequel l'exécution des instructions exécutables par machine conduit en outre le processeur à :
- recevoir (600) des données de traitement de radiothérapie (530) qui décrivent au moins partiellement la région d'intérêt ; et
- calculer (602) des instructions de commande de radiothérapie (532) pour commander l'unité de radiothérapie en utilisant au moins l'image TDM synthétique et les données de traitement de radiothérapie.

11. Système médical selon la revendication 8, 9 ou 10, dans lequel le système médical comprend en outre un système de tomodensitométrie d'émission (702), dans lequel l'exécution des instructions exécutables par machine conduit en outre le processeur à :
- commander (800) le système de tomodensitométrie d'émission pour acquérir des données d'émission de rayons gamma (712),
- calculer (802) une carte d'absorption de rayons gamma (714) en utilisant l'image TDM synthétique, et
- reconstruire (804) une image de tomodensitométrie d'émission (716) en utilisant au moins les données d'émission de rayons gamma et la carte d'absorption de rayons gamma.

12. Système médical selon l'une quelconque des revendications 8 à 11, dans lequel le système médical comprend en outre une unité d'absorptiométrie biénergétique à rayons X (902), dans lequel les données de traitement de radiothérapie comprennent une image d'absorptiométrie biénergétique à rayons X (914), et dans lequel le processeur reçoit les données de densité de minéralisation osseuse à partir de l'unité d'absorptiométrie biénergétique à rayons X.

13. Système médical selon la revendication 12, dans lequel le calcul de la valeur Hounsfield d'os cortical comprend la détermination d'une valeur de mesure de densité osseuse par absorptiométrie biénergétique à rayons X à partir de l'image d'absorptiométrie biénergétique à rayons X, dans lequel la valeur Hounsfield d'os cortical est déterminée par application d'une fonction de transfert empirique à la valeur de mesure de densité osseuse par absorptiométrie biénergétique à rayons X.

14. Système médical selon la revendication 12, dans lequel le calcul de la valeur Hounsfield d'os cortical comprend ;
- la localisation d'une région d'os cortical dans l'image d'absorptiométrie biénergétique à rayons X par segmentation de l'image d'absorptiométrie biénergétique à rayons X ;
- la détermination d'une densité d'os cortical à partir de la région d'os cortical ; et
- l'attribution de la valeur Hounsfield d'os cortical en utilisant la densité d'os cortical et une table de conversion.

15. Système médical selon l'une quelconque des revendications 8 à 11, dans lequel le système médical comprend en outre une unité de tomodensitométrie quantitative (902), dans lequel le processeur reçoit les données de densité de minéralisation osseuse à partir de l'unité de tomodensitométrie quantitative, dans lequel les données de densité de minéralisation osseuse comprennent une image de tomodensitométrie quantitative (914), dans lequel l'exécution des instructions exécutables par machine conduit le processeur à localiser une région d'os cortical dans l'image de tomodensitométrie quantitative par segmentation de l'image de tomodensitométrie quantitative, dans lequel l'exécution des instructions exécutables par machine conduit le processeur à attribuer la valeur Hounsfield d'os cortical en utilisant la région d'os cortical dans l'image de tomodensitométrie quantitative.
